# EUROPÄISCHE PATENTSCHRIFT

(11) **EP 0 812 866 B1**
(45) Veröffentlichungstag und Bekanntmachung des Hinweises auf die Patenterteilung: **15.10.2003**
(21) Anmeldenummer: 97109341.4
(22) Anmeldetag: 10.06.1997
(51) Int. Cl.: C08G 18/20, C08G 18/10, C07D 413/12

(54) **Lagerstabile, feuchtigkeitshärtende Klebstoffmasse**
Storage stable moisture-curing adhesive composition
Composition adhésive, stable au stockage et durcissable à l'humidité

(30) Priorität: 12.06.1996 CH 146496
(43) Veröffentlichungstag der Anmeldung: 17.12.1997
(73) Patentinhaber: Sika Schweiz AG, 8064 Zürich (CH)
(72) Erfinder: Merz, Peter W.,Dr., 8832 Wollerau (CH); Dux, Roland, Dr., CH-8957 Spreitenbach (CH); Grichting, Didier, 8044 Zürich (CH)
(74) Vertreter: Isler, Jörg

(56) Entgegenhaltungen:
- DE-A- 2 624 016
- DE-A- 4 327 498
- US-A- 4 780 520
- US-A- 5 195 946

## Beschreibung

### Technisches Gebiet

Die vorliegende Erfindung betrifft einen Katalysator, insbesonders für die Verwendung in Polyurethan (PU)-Systemen und speziell für die Verwendung mit feuchtigkeitsreaktiven Polyurethanen (PU), seine Herstellung und seine Verwendung. Insbesonders betrifft die Erfindung einen Katalysator, der sich für den Einsatz in unter Feuchtigkeitsausschluss lagerstabilen Klebe-, Dichtungs-, Beschichtungsmassen und Voranstrichen auf der Basis von feuchtigkeitsreaktiven Polyurethanen (PU) eignet, welche schnell in Gegenwart von Feuchtigkeit härten.

### Stand der Technik

Klebe-, Dichtungs-, Beschichtungsmassen und Voranstriche auf der Basis von Polyurethanprepolymeren, die unter Einwirkung von Feuchtigkeit aushärten, sind bekannt und werden in breitem Umfange zum Beschichten, Verbinden und Abdichten von Werkstoffen, beispielsweise Kunststoff, Glas, Keramik, lackierte Bleche, Metalle, Holz, Beton und andere Untergründe, eingesetzt.

Solche Massen sind vorteilhafterweise lösungsmittelarm oder -frei und enthalten Isocyanatgruppen enthaltende Prepolymere, welche in bekannter Weise dadurch hergestellt werden, dass zwei- oder mehrfunktionelle Polyole mit einem Überschuss an Di- oder Polyisocyanat umgesetzt werden, wobei der Monomergehalt in der Gesamtformulierung möglichst niedrig ist, d.h. kleiner 1 % insbesondere kleiner 0.5 %.

In Gegenwart von z.B. Feuchtigkeit reagieren die Isocyanatgruppen der Monomeren sowie die an den Kettenenden des Polyurethan (PU) -Prepolymeren sitzenden Isocyanatgruppen mit Wasser unter Bildung einer instabilen Carbamidsäuregruppe, die sich spontan in Amin und Kohlendioxid spaltet. Diese Amingruppe reagiert in der Folge schnell mit einer weiteren Isocyanatgruppe, wobei Harnstoffgruppen ausgebildet werden. Diese Vernetzungsreaktion bewirkt ein Molekülwachstum und führt zu einer harten oder zähelastischen Masse, die für Klebe-, Dichtungs- und Beschichtungszwecke geeignet ist.

Durch Zugabe von Katalysatoren kann die Reaktion Wasser mit Isocyanatgruppen beschleunigt werden. Bekannte Katalysatoren sind Titanate, Organometall-Verbindungen wie z.B. Zinn- oder Bleiverbindungen, welche auch mit andern Katalysatoren im speziellen mit tertiären Aminen kombiniert werden können. Im allgemeinen werden die Katalysatoren in Mengen von bis zu 2 % bezogen auf die Gesamtformulierung eingesetzt.

Bei Verwendung von grossen Katalysatormengen zwecks rascher Durchhärtung wird einerseits die Stabilität des PU-Systems beeinträchtigt, was eine Applikation nach kurzer Lagerungszeit aufgrund des Viskositätsanstieges nicht mehr möglich macht. Andererseits wird die Temperaturstabilität der gehärteten Masse aufgrund der Depolymerisation herabgesetzt. Anzustreben ist ein applikationsfähiges PU-System nach einer Lagerungszeit von mehr als 6 Monaten.

US-Patent Nr. 4780520 beschreibt die Verwendung von Dimorpholinodiethylether (DMDEE) als Katalysator, um ein lagerstabiles und schnell vernetzendes PU-System zu formulieren, wobei DMDEE in einer Menge von 0.2 bis 1.75 % bezogen auf die Gesamtformulierung vorliegt.

GB-Patentanmeldung Nr. 2231879 zeigt, dass die Verwendung von 0.2 bis 2 % Tetramethyl-DMDEE ebenfalls ein lagerstabiles PU-System ermöglicht, wobei der Festigkeitsaufbau bei niedrigen Temperaturen und Feuchte, d.h. bei 5 °C und 50 % rel. Feuchte, im Vergleich zu mit DMDEE katalysierten PU-Systemen rascher erfolgt.

In DE 43 27 498 wird ein orthopädisches Verbandsmaterial offenbart, das ein Polyurethan-Prepolymer und einen Mischkatalysator aus α-Morpholinoethoxy-ethoxy-β-morpholino-ethan und wasserfreiem Kaliumcarbonat enthält, und in US 5,195,946 werden Morpholinogruppen enthaltende Katalysatoren für die Verwendung in wasseraktivierten Polyurethan-Bandagen für die Verwendung als orthopädische Gussform beschrieben.

In US 5,195,946 werden wasseraktivierte Polyurethan-Bandagen beschrieben, welche ein Glas- oder synthetische Fasergewebe enthalten, welche mit einem wasseraktivierten Polyurethan Prepolymer überschichtet sind, wobei dieses Prepolymer ein aromatisches Isocyanat, eine Polyhydroxy-Verbindung sowie einen Katalysator enthalten. Der hierbei verwendete Katalysator ist α-(morpholinopolyethoxy)-β-morpholinoethan oder eine Mischung davon mit bis-(2-dimethylaminoethyl)ether.

Im US-Patent Nr. 4705840 werden 2,2'-Dimorpholinylalkylether aufgezeigt, welche im Vergleich zum DMDEE bei halber Konzentration, d.h. bei 5 mol % die gleiche Frühfestigkeit, d.h. 7.5 Minuten nach Anlegen einer orthopädischen Bandage, bei 24 °C und 55 % rel. Feuchte zeigen.

Die Verwendung der oben erwähnten Katalysatoren für die Formulierung von schnellhärtenden PU-Systemen mit raschem Festigkeitsaufbau hat den Nachteil, dass die Fügezeit auf unter 8 bis 10 Minuten reduziert wird. Die Fügezeit, welche auch als Offenliegezeit bezeichnet werden kann, wird als die Zeit zwischen der Applikation und dem Fügen definiert, bei welcher eine gute Haftung gewährleistet ist. Die Hautbildungszeit ist erfahrungsgemäss kleiner oder gleich der Fügezeit und stellt für eine ungefähre Bestimmung der Fügezeit eine effiziente Prüfmethode dar (siehe Beispiele).

### Zusammenfassung der Erfindung

Die vorliegende Erfindung hat sich der Aufgabe gewidmet, Katalysatoren auf der Basis von Morpholin für die Herstellung von schnellen, lagerfähigen PU-Systemen zur Verfügung zu stellen, welche bei tiefen Temperaturen und tiefer Luftfeuchtigkeit (z.B. 5 °C und 80 % rel. Feuchte bzw. 23 °C und 20% rel. Feuchte) einen raschen Festigkeitsaufbau mit im Vergleich zum Stand der Technik verlängerter Fügezeit, d.h. grösser 10 Minuten, besitzen, und andererseits eine gute Temperaturstabilität aufweisen.

Dies wurde mit den erfindungsgemässen Katalysatoren der folgenden Formel erreicht 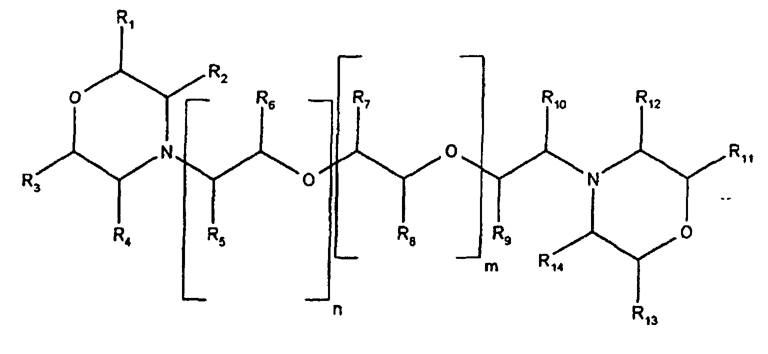 wobei n+m zwischen 2 und 10 liegt und R₁, R₂, R₃, R₄, R₅, R₆, R₇, R₈, R₉, R₁₀, R₁₁, R₁₂, R₁₃, R₁₄ unabhängig voneinander je Wasserstoff oder eine Alkylgruppe, insbesondere eine Methylgruppe oder eine Ethylgruppe, bedeuten.

Die erfindungsgemässen Katalysatoren erhöhen primär die Reaktivität der Isocyanatgruppen und sind deshalb sowohl für 1-Komponenten(1K)- wie auch 2-Komponenten (2K)-Systeme, die mit Wasser oder Härtern auf Basis von z.B. reaktiven OH- oder NH₂-Gruppen aushärten, geeignet. Sie können alleine sowie in Kombination untereinander oder in Kombination mit bekannten Katalysatoren z.B. auf Basis metallorganischer Zinn- und/oder Titankatalysatoren (vgl. vorne) verwendet werden.

Die erfindungsgemässen Katalysatoren sind insbesonders auch für feuchtigkeitshärtende 1K-Systeme gut geeignet.

Die Wirkungsweise dieser oben beschriebenen Morpholin-Derivate in feuchtigkeitshärtenden Systemen beruht wahrscheinlich darin, dass einerseits durch den hydrophilen Teil - ähnlich eines Kronenethers - Wasser in die Matrix und somit in die Nähe der reaktiven Isocyanatgruppen transportiert wird und andererseits die beiden tertiären Amine katalytisch helfen, das sich somit in unmittelbare Nähe befindliche Wasser zur Reaktion mit der Isocyanatgruppe zu bringen. Dadurch sind diese Katalysatoren sehr geeignet für die Verwendung in 1K-Systemen. Diese katalytische Wirkung involviert möglicherweise *'einen Protontransfer zum Amidstickstoff der Isocayanatgruppierung'* (Lit.: Herlinger Heinz, Habilitationsschrift 'Struktur und Reaktivität der Isocyanate' Universität Stuttgart 1970, Seite 24.). Nach der Isocyanat-Wasser Reaktion entsteht einerseits Kohlendioxid, und andererseits wird der Katalysator, das Morpholin-Derivat, wieder freigesetzt. Wie bereits oben erwähnt, handelt es sich bei dieser Erklärung der Wirkungsweise um eine wahrscheinliche Erklärung. Diese soll in keiner Weise als den Gegenstand der Erfindung einschränkend gewertet werden.

In einer besonders günstigen Ausführungsform ist die Hydrophilie des Morpholin-Derivates erhöht und dies wird durch einen Einbau eines Polyethylenoxides zwischen zwei Morpholingruppen erreicht. Der wasserabsorbierende Charakter der erfindungsgemässen Katalysatoren und somit der Diffusionsgradient des PU-Systems, ist derart, dass das Wasser aus der Umgebungsfeuchte in die PU-Masse gelangt, bevor es von den an der Oberfläche vorhandenen Isocyanatgruppen abgefangen wird. Dadurch wird die Hautbildungszeit etwas hinausgezögert, und die Fügezeit wird bei gleichzeitig rasch erfolgender Festigkeitsentwicklung verlängert. Diese Eigenschaftskombination ist für den Anwender von grossem Vorteil. Ferner werden die auf der Substratoberfläche vorliegenden Wasserspuren aufgrund des hygroskopischen Charakters der PU-Masse von der Zwischenschicht, Klebstoff zum Untergrund, wegtransportiert. Dadurch wird keine sofortige Vernetzung erreicht, was zu einer optimaleren Benetzung zum Untergrund und somit zu besseren Haftungseigenschaften führt.

Die erfindungsgemässen PU-Massen, die mit den erfindungsgemässen Katalysatoren katalysiert sind, können wegen ihrer hervorragenden Stabilität bei erhöhter Temperaturen, d.h. bis 95 °C, verarbeitet werden und ermöglichen somit auch die Herstellung von lagerfähigen, feuchtigkeitsreaktiven Schmelzklebstoffen, welche von allen einkomponentig härtenden Systemen aufgrund des durch Abkühlung nach der Applikation erfolgten Viskositätsanstieges und des mit Feuchtigkeit rasch härtenden Bindemittelsystems den grössten Festigkeitsaufbau offerieren.

Die erfindungsgemässen PU-Zusammensetzungen sind insbesonders geeignet als Klebstoffe, Dichtstoffe, Beschichtungsmaterialien oder Voranstriche. Sie weisen bei rasch erfolgendem Festigkeitsaufbau eine im Vergleich zum Stand der Technik verzögerte Hautbildungszeit auf und offieren somit eine verlängerte Fügezeit. Sie sind für die Anwendung auf Metall-, Glas-, Keramik-, Holz-, zementösen und Kunststoff-Substraten geeignet.

### Ausführliche Beschreibung der Erfindung

Der erfindungsgemässe Katalysator ist eine Morpholingruppen-enthaltende Verbindung der allgemeinen Formel 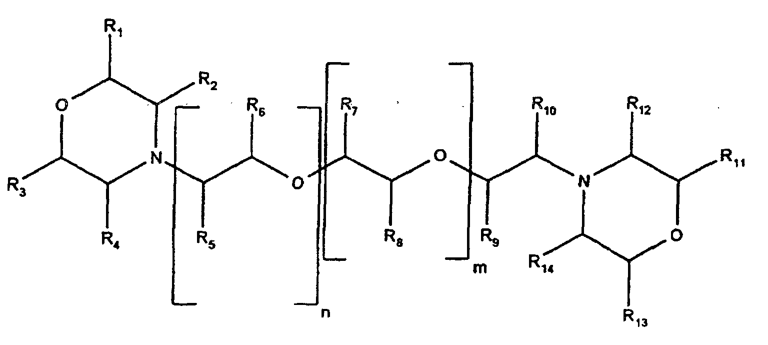 worin n+m zwischen 2 und 10 liegt und R₁ bis R₁₄ unabhängig voneinander je Wasserstoff oder eine Alkylgruppe, insbesonders eine Methylgruppe oder eine Ethylgruppe, bedeuten. In bevorzugten Katalysatoren liegt die Summe von n+m zwischen 2 und 5. Spezielle Katalysatoren, die relativ leicht herstellbar sind und gute Wirkung zeigen, sind beispielsweise jene mit
- R₁ bis R₁₄ = Wasserstoff
- R₁ bis R₄ und R₉ bis R₁₄ = Wasserstoff und R₅ oder R₆ und/oder R₇ oder R₈ = Methylgruppen, wobei jene R₅, R₆, R₇ und R₈, die keine Methylgruppe sind, Wasserstoff bedeuten.
- R₁, R₃, R₁₁ und R₁₃ = Methylgruppen und R₂, R₄ bis R₁₀, R₁₂ und R₁₄ = Wasserstoff.
   Die erfindungsgemässen Katalysatoren können beispielsweise gemäss der kanadischen Patentanmeldung CA 2103730 von Miles Inc., USA (1992) hergestellt werden.
   Sie eignen sich insbesonders für die Verwendung in Isocyanatgruppen enthaltenden 1-Komponenten (1K)-und 2-Komponenten (2K)-PU-Systemen. Insbesonders eignen sich die erfindungsgemässen Katalysatoren sehr gut für die Verwendung in 1K-Systemen.
   Die Isocyanatgruppen enthaltenden PU-Prepolymere, die in den erfindungsgemässen Systemen als die Hauptkomponente vorliegen, sind Reaktionsprodukte von Isocyanatgruppen enthaltenden Substanzen mit irgendeiner mit Isocyanatgruppen reaktiven Verbindung, wie z.B. aliphatische oder aromatische Polyol-, Polyamin- oder Polymercaptogruppen enthaltende Verbindungen, wobei die Umsetzung in bekannter Weise bei Temperaturen um 80 °C und gegebenenfalls in Gegenwart eines Katalysators, z.B. Dibutylzinndilaurat meistens stöchiometrisch, d.h. pro H-aktive Gruppe ein mindestens 2 Isocyanatgruppen enthaltendes Monomer, erfolgt.
   Üblicherweise werden Polyole mit einer Funktionalität zwischen 1.5 und 3 sowie einem Molekulargewicht zwischen 400 und 10000, vorzugsweise im Bereich von etwa 1000 und 6000 verwendet. Solche Polyole sind z.B. Polyalkylenpolyole (z.B. Polyethylenoxide, Polypropylenoxide, Polybutylenoxide, Polytetrahydrofurane), Polycarbonate, Polycaprolactone, Polyester,etc..
   Die Isocyanatgruppen enthaltenden Monomere können aliphatisch, cycloaliphatisch oder aromatisch sein, wie z.B. 4,4'-Diphenylmethandiisocyanat, 2,4-Toluoldiisocyanat, Hexamethylendiisocyanat, Isophorondiisocyanat, Perhydro-2,4'-diphenylmethandiisocyanat etc.
   Die oben beschriebenen PU-Prepolymere haben bevorzugt einen freien Isocyanatgehalt im Bereich von 1 bis 3 % und liegen normalerweise zwischen 20 bis 60 %, insbesondere zwischen 20 und 50 %, bezogen auf die Gesamtformulierung vor.
   Die PU-Systeme der vorliegenden Erfindung bestehen aus mindestens einem PU-Prepolymer und dem erfindungsgemässen Härtungskatalysator und können gegebenenfalls zusätzlich übliche Additive und Hilfstoffe enthalten, beispielsweise Weichmacher, Füllstoffe, latente Härter, Haftungsverbesserer, Farbstoffe, Pigmente, UV-Absorptionsmittel, Stabilisatoren, Antioxidantien, oberflächenaktive Additive, Flammhemmer, fungistatisch aktive Substanzen etc. Die Art und Menge dieser Zusatzstoffe richtet sich nach dem Verwendungszweck der erfindungsgemässen Massen.
   Die Menge der erfindungsgemässen Isocyanat / Wasser-Härtungskatalysatoren sollte im allgemeinen 0.1 bis 2 % (Gewichtsprozent), insbesondere 0.4 bis 1 %, bezogen auf die Gesamtformulierung betragen. Während bei einer Menge unterhalb von 0.1 % der gewünschte Härtungseffekt nicht erzielt wird, wird bei einer Menge oberhalb von 2 % die Lagerstabilität des PU-Systems beeinträchtigt.
   Wenn notwendig, können die erfindungsgemässen Katalysatoren mit andern konventionellen Katalysatoren, z.B. Organometall- oder tertiäre Amin-Katalysatoren kombiniert werden.
   Bei der Herstellung erfindungsgemässer 1K-Massen ist dafür zu sorgen, dass keine Feuchtigkeit eingebracht wird. Alle verwendeten Komponenten sollten möglichst frei von Wasser sein, und es ist zweckmässig den PU-Systemen wasserbindende oder mit Wasser reagierende Stoffe zu zumischen, wie z.B. Calciumoxid, Molekularsieb, monofunktionelle Isocyanatgruppen enthaltende Verbindungen, Orthoameisensäureester etc.. Die Konfektionierung erfolgt unter Feuchtigkeitsausschluss in bekannter Weise z.B. in Kartuschen, Fass etc..
   Die erfindungsgemässen PU-Systeme können je nach Anforderung für das Verbinden, Abdichten oder Beschichten verwendet werden und finden im Baubereich sowie in der Industrie z.B. im Bereich der Fahrzeugherstellung, Marine etc. zahlreiche Einsatzmöglichkeiten, wobei auf verschiedensten Materialien, wie z.B. Glas, Keramik, Kunststoffe, PU-Elastomer, Metalle, lackierte Metalle, appliziert wird. Gegebenenfalls muss eine Vorbehandlung mit einem Primervoranstrich durchgeführt werden, um eine optimale Haftung zu erlangen.
   Die folgenden Ausführungen am Beispiel 1K Klebstoffe sollen die Erfindung weiter veranschaulichen. Diese sind in keiner Weise als den Umfang der Erfindung in irgendeiner Form beschränkend anzusehen.

### Beispiele

### Untersuchung der Lagerstabilität der Klebstoffzusammensetzungen:

Die Viskosität der Klebstoffzusammensetzung wurde mittels Extrusion einer Kartusche bei 23 °C und einem Druck von 6 bar durch eine 3 mm-Düse bestimmt, was zu einem Wert in Gramm pro Minute führte. Diese Messung wurde nach Lagerung bei Raumtemperatur während 7 Tagen (=> ursprüngliche Extrusionsrate), bzw. 1 Monat und 3 Monate, sowie nach Hitzealterung bei 60 °C während 7 Tagen durchgeführt. Zusätzlich wurde jeweils die Hautbildungszeit bestimmt, um den Einfluss der Lagerbedingungen auf die Reaktivität des Klebstoffes, respektive des Härtungskatalysators zu untersuchen.

### Lagerung der Probekörper:

Die Lagerung der Probekörper erfolgte bei zwei verschiedenen klimatischen Verhältnisse, um deren Einfluss auf die Offenliegezeit bzw. den Festigkeitsaufbau zu eruieren:
Klima I: 23 °C und 50 % rel. Feuchte (Standardklima)
Klima II: 5 °C und 80 % rel. Feuchte.

### Bestimmung der Hautbildungszeit (HBZ):

Die Hautbildungszeit ist die Zeit nach der Applikation bis zur Klebfreiheit.

### Bestimmung der Frühfestigkeit:

Die Entwicklung des Festigkeitsaufbaus wurde mittels Zugscherprobekörper bestehend aus zwei Glasplatten nach DIN 53504 (Zuggeschwindigkeit: 200 mm/min, Klebstoffschichtdicke: 5 mm) nach Lagerung der Probekörper jeweils unter den zwei oben definierten klimatischen Bedingungen bestimmt, wobei die Messung jeweils nach 30 Min., 60 Min., 90 Min. bzw. 3 Stunden erfolgte. Der Wert der Zugscherfestigkeit (ZSF) wird in N/cm² angegeben.

### Katalysatoren:

Folgende Katalysatoren wurden im Vergleich geprüft:
1) DMDEE (2,2'-Dimorpholinoethylether) von Nitroil, DE
2) TMDMDEE (Tetramethyl-DMDEE) von Nitroil, DE
3) DMPEG200 (Dimorpholino-polyethylenoxidglykol) (n+m ca. 3)

Während die beiden Katalysatoren 1) und 2) zum Stand der Technik gehören, stellt 3) einen erfinderischen Katalysator dar (siehe Formel, Herstellung vgl. hinten).

### Klebstoffformulierung:

Die Wirkungsweise der Katalysatoren wurde in einer Standard-Formulierung untersucht, wobei diese, neben Russ und Kreide, auf einem Prepolymer, bestehend aus einem trifunktionellen Polyetherpolyol mit einem Molekulargewicht von ca. 4500 und einem aromatischen Isocyanatgruppen enthaltenden Monomer, MDI (Methylen-4,4'-diphenyldiisocyanat), aufgebaut ist. Die Katalysatormenge wurde so berechnet, dass der Morpholingehalt 0.4 Aequivalenzprozente betrug. Dies entspricht für DMDEE etwa 0.5, für TMDMDEE etwa 0.6 und für den erfindungsgemässen Katalysator DMPEG200 etwa 0.8 Gewichtsprozent bezogen auf die Gesamtformulierung der Klebstoffmasse.

### Zubereitung des erfindungsgemässen Katalysators DMPEG200 (gemäss der kanadischen Patentanmeldung CA 2103730 / Miles Inc. USA / 1992)

### A) Herstellung des Dimesylates von PEG200

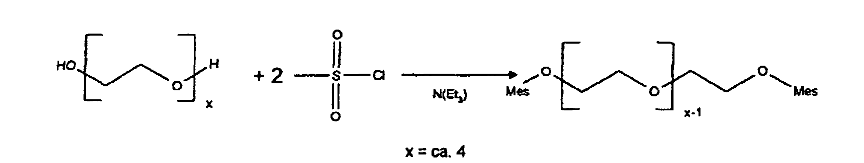

11.41 g Polyethylenglycol 200 (Fluka, pract.) wird mit 13.1g Triethylamin und 22 ml Methylenchlorid (Fluka, puriss.) in einem 250ml-3-Halskolben (Rückflusskühler und Tropftrichter aufgesetzt, sowie Thermometer) vorgelegt und mit Stickstoff gespült. Dann wird das Mesylchlorid aus einem 25 ml Tropftrichter unter Inertatmosphäre und intensivem Rühren mittels Magnetrührer langsam zugetropft. Weil die stattfindende Reaktion sehr exothermisch ist, wird der Kolben mit Eiswasser gekühlt und die Tropfgeschwindigkeit derart geregelt, dass die Temperatur nicht über 25°C steigt. Bei der Reaktion fällt ein weiss/gelber Niederschlag aus. Nach dem Ende des Zutropfens wird der Tropftrichter mit 20 ml Methylenchlorid gespült, das in die Reaktionsmischung getropft wird. Nach dem Entfernen des Eisbades wird die Mischung bei Raumtemperatur während 1.5 Std. gerührt und anschliessend mit einer konzentrierten Natriumhydroxidlösung (5.1 g NaOH (Fluka 71691) in 20 ml Wasser) vorsichtig neutralisiert. Der Niederschlag löst sich hierbei auf und eine gelbe Phase resultiert. Anschliessend wird eine weitere Stunde bei RT gerührt und dann die Phase am Wasserstrahlvaccum eingeengt [hierbei wird die Wasserbadtemperatur langsam auf 70 °C erhitzt (den Auffangkolben sollte man mit Wasser/Eis kühlen, um das Dichlormethan der Wiederverwertung zuführen zu können)], wobei wiederum ein gelber Niederschlag ausfällt. Eine weitere Reinigung dieses Zwischenproduktes wurde nicht vorgenommen. Das so erhaltene Zwischenprodukt wurde sofort für die weitere Umsetzung verwendet.

### B) Herstellung von DMPEG200

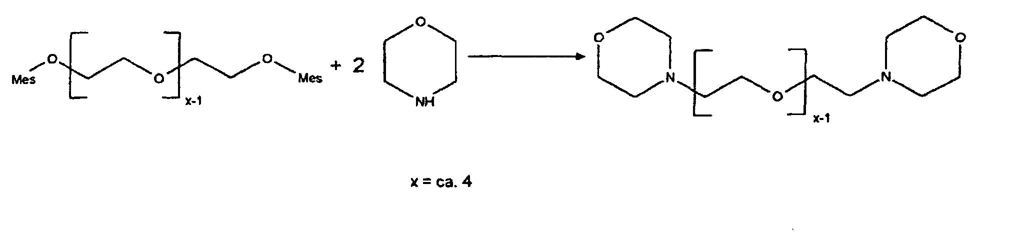

Der milchige Rückstand des Zwischenproduktes wird mit 20 ml Methylenchlorid verdünnt und mit 15.87g Morpholin, 112 ml Isopropylalkohol und 20.1 g wasserfreiem Natriumcarbonat (Fluka puriss F71350)(im Ofen über Nacht bei 130°C getrocknet) versetzt. Die Reaktionsmischung wird nun auf Rückfluss erhitzt (Badtemp. ≈ 85°C) und bei dieser Temperatur während etwa 5 Stunden belassen. Das Reaktionsprodukt wird über eine Glasfritte (Porosität 4) abfiltriert und vom weissen Rückstand befreit, aus welchem sich noch kleinere Mengen des Produktes durch Extraktion mit Ether isolieren liessen. Das leicht gelbliche Filtrat wird an der Wasserstrahlpumpe eingeengt und dann auf 80°C Badtemperatur erhitzt und während einer Stunde so belassen. Hierbei fällt eine geringe Menge Festkörper erneut aus. Dieser Rückstand wird nun während 3 Stunden am Rotationspumpenvakuum von flüchtigen Teilen befreit.

Es werden anschliessend 100 ml Diethylether (Fluka purum, F 31700) (*PEROXIDFREI* !) hinzu gegeben und während 15 Minuten gerührt und anschliessend über eine Glassfritte (Porosität 4) filtriert. Das resultierende Filtrat wird unter Destillation des Ethers bei normalem Druck eingeengt und anschliessend unter Wasserstrahlvakuum während 1 Std. bei 80°C Badtemperatur getrocknet. Die zurückbleibende Lösung wird schliesslich während 2 Stunden am Rotationspumpenvakuum (P≈10^{-1.5}mbar) vollständig getrocknet. Vom so gereinigten Produkt DMPEG200 erhält man ca. 14.7 g, was einer Ausbeute von ca. 75% entspricht. Es enthält weniger als ≈ 0.04% Wasser (Bestimmung durch Karl-Fischer-Titration).

### Ergebnis der Vergleichsprüfung:

Die Bewertung erfolgte im Vergleich zum mit DMDEE-katalysierten PU-Klebstoff und zwar bedeutet:
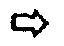 ⇒ gleiche Performance
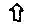 verlängerte Hautbildungszeit / schnellerer Festigkeitsaufbau
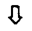 verkürzte Hautbildungszeit / langsamerer Festigkeitsaufbau

### A) HBZ und Festigkeitsentwicklung im Klima I (23 °C / 50 % r.H.)

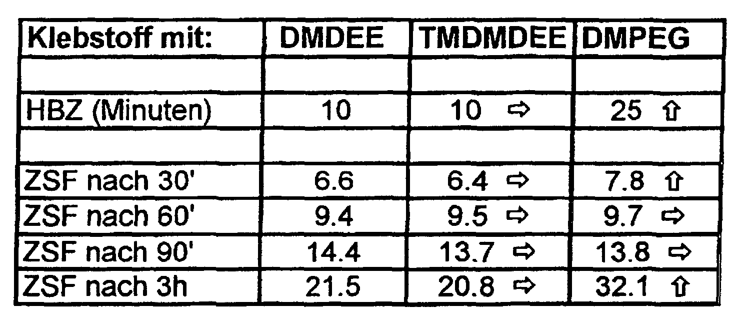

### B) HBZ und Festigkeitsentwicklung im Klima II (5 °C / 80 % r.H.)

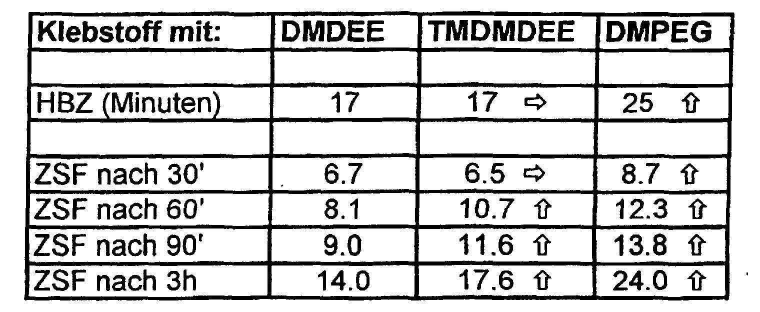

### C) Lagerstabilitätsuntersuchungen

- Die Bewertung erfolgte durch Angabe der prozentualen Aenderung zum ursprünglichen Wert. Der mit DMDEE-katalysierten PU-Klebstoff weist aufgrund Praxiserfahrung eine zufriedenstellende Lagerungsstabilität auf.

### Auspressmenge

| **Klebstoff mit:** | **DMDEE** | **TMDMDEE** | **DMPEG** |
|---|---|---|---|
| | | | |
| 1 Mt bei RT | < 20 % | < 20 % | < 20 % |
| 3 Mt bei RT | < 40 % | < 40 % | < 40 % |

### Hautbildungszeit

| **Klebstoff mit:** | **DMDEE** | **TMDMDEE** | **DMPEG** |
|---|---|---|---|
| | | | |
| 1 Mt bei RT | < 10 % | < 10 % | < 10 % |
| 3 Mt bei RT | < 10 % | < 10 % | < 10 % |
| | | | |

## Patentansprüche

1. Verwendung einer Ein-Komponenten-Polyurethan-Zusammensetzung enthaltend oder bestehend aus mindestens einem Prepolymer auf Basis aromatischen oder aliphatischen Isocyanats und mindestens einem morpholingruppen enthaltenden Katalysator der allgemeinen Formel 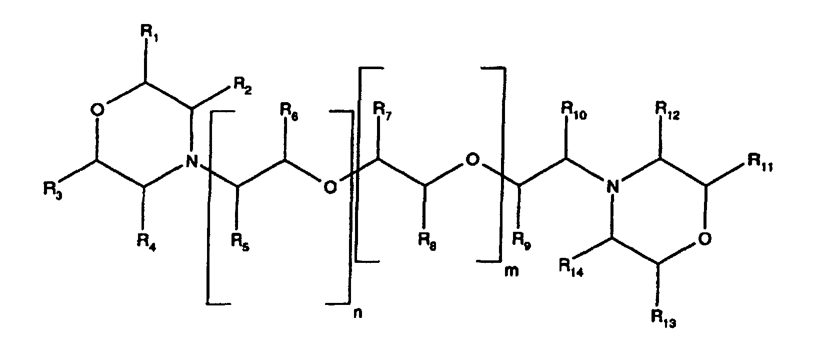 worin die Summe von n+m zwischen 2 und 10 liegt und R₁, R₂, R₃, R₄, R₅, R₆, R₇, R₈, R₉, R₁₀, R₁₁, R₁₂, R₁₃, R₁₄ unabhängig voneinander je Wasserstoff oder eine Alkylgruppe bedeuten, als durch Wasser aus der Umgebungsfeuchte auszuhärtender Klebstoff, Dichtstoff, Beschichtungsmaterial und/oder Voranstrich.

2. Verwendung gemäss Anspruch 1, **dadurch gekennzeichnet, dass** im Katalysator die Summe von n+m zwischen 2 und 5 liegt.

3. Verwendung gemäss Anspruch 1 oder 2, **dadurch gekennzeichnet, dass** im Katalysator R₁ bis R₁₄ Wasserstoff sind.

4. Verwendung gemäss Anspruch 1 oder 2, **dadurch gekennzeichnet, dass** die R₁ bis R₁₄, die eine Alkylgruppe bedeuten, Methyl- oder Ethylgruppen sind.

5. Verwendung gemäss Anspruch 4, **dadurch gekennzeichnet, dass** im Katalysator R₁, R₂, R₃, R₄, R₉, R₁₀, R₁₁, R₁₂, R₁₃ und R₁₄ Wasserstoff und R₅ oder R₆ und/oder R₇ oder R₈ Methylgruppen sind, wobei die R₅, R₆, R₇ und R₈, die nicht eine Methylgruppe sind, Wasserstoff bedeuten.

6. Verwendung gemäss Anspruch 4, **dadurch gekennzeichnet, dass** im Katalysator R₁, R₃, R₁₁ und R₁₃ Methylgruppen und R₂, R₄, R₅, R₆, R₇, R₈, R₉, R₁₀, R₁₂ und R₁₄ Wasserstoff sind.

7. Verwendung gemäss einem der Ansprüche 1 bis 6, **dadurch gekennzeichnet, dass** im Katalysator die Summe von n+m ca. 3 beträgt.

8. Verwendung gemäss einem der Ansprüche 1 bis 7, **dadurch gekennzeichnet, dass** das Prepolymer das Reaktionsprodukt von Isocyanatgruppen enthaltenden Substanzen mit Polyalkylenpolyolen einer Funktionalität zwischen 1.5 und 3 und einem Molekulargewicht zwischen 400 und 10'000 ist.

9. Verwendung gemäss einem der Ansprüche 1 bis 8, **dadurch gekennzeichnet, dass** der Katalysator in der Polyurethan-Zusammensetzung in einer Menge von 0,1 bis 2 Gew.-% bezogen auf die Gesamtformulierung vorliegt.

## Claims

1. The use of a single-component polyurethane composition comprising or consisting of at least one prepolymer on the basis of aromatic or aliphatic isocyanate and at least one morpholine groups comprising catalyst of the formula 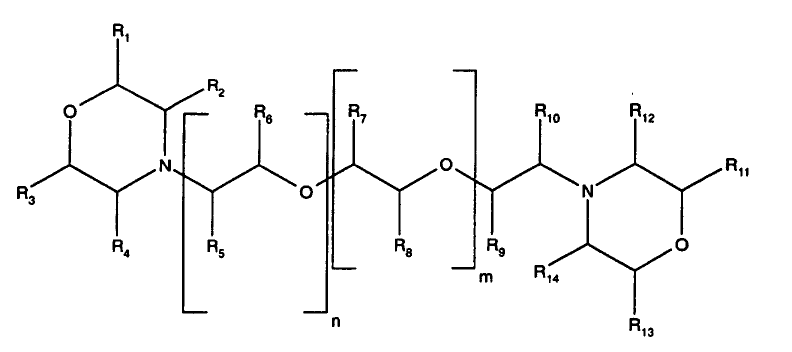 wherein the sum of n + m is between 2 and 10 and R₁, R₂, R₃, R₄, R₅, R₆, R₇, R₈, R₉, R₁₀, R_{11,} R₁₂, R₁₃ and R₁₄ are independently of each other hydrogen or an alkyl group as adhesive, sealant, coating material and/or primer to be cured by water from the ambient humidity.

2. The use of claim 1, **characterized in that** in the catalyst the sum of n + m is between 2 and 5.

3. The use of claim 1 or 2, **characterized in that** in the catalyst R₁ to R₁₄ are hydrogen.

4. The use of claim 1 or 2, **characterized in that** those of R₁ to R₁₄ which are an alkyl group are methyl or ethyl groups.

5. The use of claim 4, **characterized in that** in the catalyst R₁, R₂, R₃, R₄, R₉, R₁₀, R₁₁, R₁₃ and R₁₄ are hydrogen and R₅ or R₆ and/or R₇ or R₈ are methyl groups, where those of R₅, R₆, R₇ and R₈ that are not a methyl group are hydrogen.

6. The use of claim 4, **characterized in that** in the catalyst R₁, R₃, R₁₁ and R₁₃ are methyl groups and R₂, R₄, R₅, R₆, R₇, R₈, R₉, R₁₀, R₁₂ and R₁₄ are hydrogen.

7. The use of one of claims 1 to 6, **characterized in that** in the catalyst the sum of n + m is approximately 3.

8. The use of one of claims 1 to 7, **characterized in that** the prepolymer is the reaction product of isocyanate groups comprising substances with polyalkylene polyols of a functionality between 1.5 and 3 and a molecular weight between 400 and 10 000.

9. The use of one of claims 1 to 8, **characterized in that** the catalyst is present in the polyurethane composition in an amount of 0.1 to 2% by weight referred to the whole formulation.

## Revendications

1. Utilisation d'une composition de polyuréthane à un composant qui contient ou qui est constituée d'au moins un pré-polymère à base d'isocyanate aromatique ou aliphatique et d'au moins un catalyseur qui contient des groupes morpholine et de formule générale : 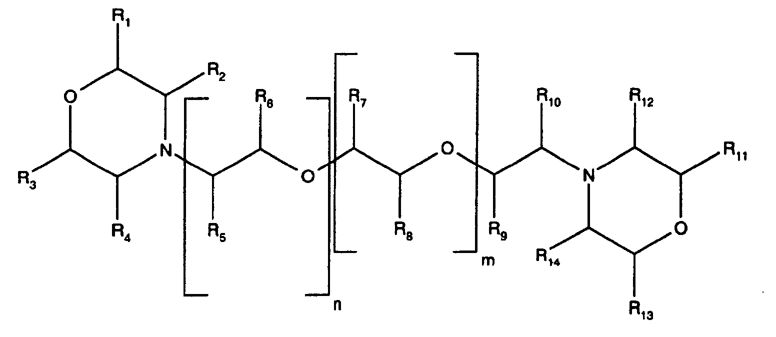 dans laquelle la somme n + m est comprise entre 2 et 10 et R₁, R₂, R₃, R₄, R₅, R₆, R₇, R₈, R₉, R₁₀, R₁₁, R₁₂, R₁₃, R₁₄ représentent chacun indépendamment l'un de l'autre l'hydrogène ou un groupe alkyle, en tant qu'adhésif, agent d'étanchéité, matériau de revêtement et/ou de couche de fond durcissant sous l'action de l'eau provenant de l'humidité environnante.

2. Utilisation selon la revendication 1, **caractérisée en ce que** dans le catalyseur, la somme n + m est comprise entre 2 et 5.

3. Utilisation selon la revendication 1 ou 2, **caractérisée en ce que** dans le catalyseur, R₁ à R₁₄ représentent l'hydrogène.

4. Utilisation selon la revendication 1 ou 2, **caractérisée en ce que** les R₁ à R₁₄ qui représentent un groupe alkyle représentent des groupes méthyle ou éthyle.

5. Utilisation selon la revendication 4, **caractérisée en ce que** dans le catalyseur, R₁, R₂, R₃, R₄, R₉, R₁₀, R₁₁, R₁₂, R₁₃ et R₁₄ représentent l'hydrogène et R₅ ou R₆ et/ou R₇ ou R₈ représentent des groupes méthyle, les R₅, R₆, R₇ et R₈ qui ne représentent pas un groupe méthyle représentant l'hydrogène.

6. Utilisation selon la revendication 4, **caractérisée en ce que** dans le catalyseur, R₁, R₃, R₁₁ et R₁₃ représentent des groupes méthyle et R₂, R₄, R₅, R₆, R₇, R₈, R₉, R₁₀, R₁₂ et R₁₄ représentent l'hydrogène.

7. Utilisation selon l'une des revendications 1 à 6, **caractérisée en ce que** dans le catalyseur, la somme n + m vaut environ 3.

8. Utilisation selon l'une des revendications 1 à 7, **caractérisée en ce que** le pré-polymère est le produit de réaction de substances qui contiennent des groupes isocyanate avec des polyalkylènes polyols dont la fonctionnalité est comprise entre 1,5 et 3 et dont le poids moléculaire est compris entre 400 et 10 000.

9. Utilisation selon l'une des revendications 1 à 8, **caractérisée en ce que** le catalyseur est présent dans la composition de polyuréthane en quantité de 0,1 à 2% en poids par rapport à la formulation totale.
